(19) Europäisches Patentamt
European Patent Office
Office européen des brevets

(11) Publication number: 0 093 484
A2

(12) # EUROPEAN PATENT APPLICATION

(21) Application number: 83300688.5

(22) Date of filing: 11.02.83

(51) Int. Cl.³: C 07 D 407/06
A 61 K 31/35

(30) Priority: 25.02.82 GB 8205547

(43) Date of publication of application: 09.11.83 Bulletin 83/45

(84) Designated Contracting States: CH DE FR GB IT LI NL

(71) Applicant: BEECHAM GROUP PLC
Beecham House Great West Road
Brentford Middlesex(GB)

(72) Inventor: O'Hanlon, Peter John
65 Green Lane
Redhill Surrey(GB)

(72) Inventor: Robertson, Fiona Mary
Flat 2, Appletree Cottage Garden Close
Givons Grove Leatherhead Surrey(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

(54) **Antibacterial monic acid derivatives.**

(57) Compounds of the formula (I):

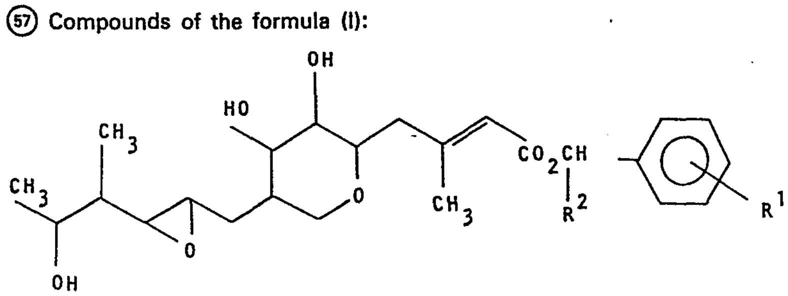

wherein $R^1$ is alkylthio, alkylsulphinyl or alkylsulphonyl, and $R^2$ is hydrogen or alkyl, are useful in treating bacterial and mycoplasmal infections.

EP 0 093 484 A2

Croydon Printing Company Ltd.

## ANTIBACTERIAL COMPOUNDS

The present invention relates to compounds having antibacterial and/or antimycoplasmal activity, to processes for their production and to their use in human and veterinary medicine.

U.K. Patent No. 1 587 059 describes a class of compounds including "monic acid A" and pharmaceutically acceptable esters thereof having the formula:

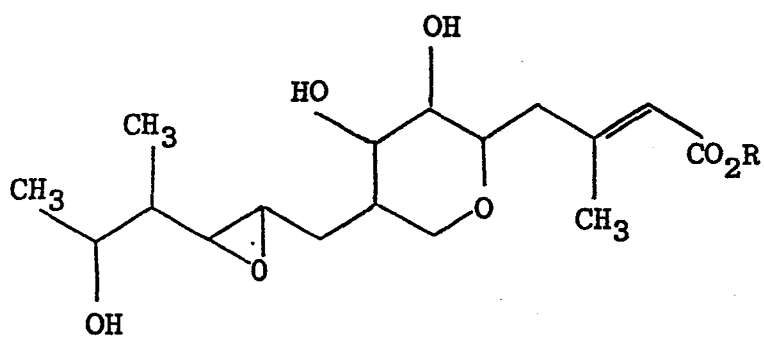

monic acid A, R=H

It has now been found that a certain narrow class of pharmaceutically acceptable monic acid A esters, falling within the above formula but not specifically disclosed in the above patent, have particularly advantageous properties including good antibacterial and/or antimycoplasmal activity.

Accordingly the present invention provides a compound of formula (I)

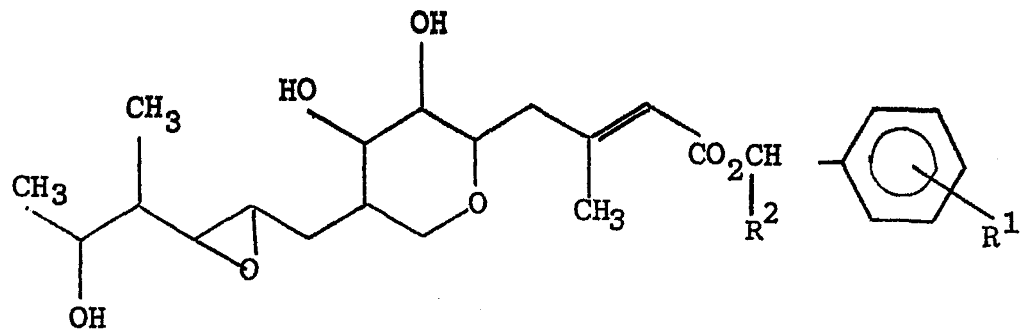

wherein $R^1$ is alkylthio, alkylsulphinyl or alkylsulphonyl, and $R^2$ is hydrogen or alkyl.

As used herein the term "alkyl" refers to groups having from 1 to 4 carbon atoms. Such groups having 3 or 4 carbon atoms may be straight, branched or cyclic.

Preferably the alkyl group of $R^1$ is methyl or ethyl, most preferably methyl. Preferably $R^2$ is hydrogen.

$R^1$ may be located at any position on the benzene ring; suitably it is at the <u>meta</u>- or <u>para</u>-position with respect to the monic acid residue, preferably at the <u>para</u>-position.

The present invention also provides a process for producing compounds of formula (I), which process comprises reacting a compound of formula (II)

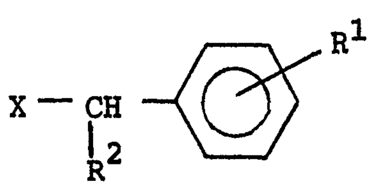

(II)

wherein X is a good leaving group with a reactive derivative of monic acid A.

As used herein the term "reactive derivative of monic acid A" refers to any compound capable of reacting with the compound of formula (II) to produce a compound of formula (I)

Suitably X is halogen, alkylsulphonate or arylsulphonate; preferably X is halogen and most preferably chlorine or bromine.

Suitably the reactive derivative of monic acid A is an alkali metal salt thereof, preferably the sodium salt.

The reaction is suitably effected at ambient or elevated temperature, preferably at about 20 to 100$^oC$, in an organic solvent such as dimethylformamide. The product may be purified by chromatographic techniques, such as column chromatography on silica gel.

Compounds of formula (I) wherein $R^1$ is alkylthio may be oxidised to the corresponding compound wherein $R^1$ is alkylsulphinyl and/or alkylsulphonyl using conventional oxidising agents. Likewise the compounds wherein $R^1$ is alkylsulphinyl may be oxidised to the corresponding alkylsulphonyl derivative.

Suitable oxidising agents include peracids, hydroperoxides, t-butyl hypochlorite, N-bromosuccinimide and m-chloroperbenzoic acid.

When certain oxidising agents are employed, it may be desirable to protect other oxidisable groups on the monic acid nucleus prior to the reaction and to remove such protection after the oxidation reaction. This may be achieved using conventional protecting groups, such as those described in European Patent Application No. 0005 006.

Such protection is not always necessary; for instance it has been found that when m-chloroperbenzoic acid is used as oxidising agent, good yields may be achieved without protection of the monic acid nucleus.

Monic acid A is readily obtainable by the methods described in U.K. Patent No. 1 587 058, and reactive derivatives thereof are produced by conventional methods.

The infections of humans against which compounds of this invention are particularly useful include venereal disease. The compounds are also effective in the treatment of respiratory infections such as bacterial bronchitis; and bacterial meningitis, non-specific urethritis and pneumonia. In animals, the compounds may be employed for the treatment of mastitis in cattle, for swine dysentery, and for mycoplasma infections in animals such as turkeys, chickens, pigs and cattle.

Some of the human and veterinary diseases either caused by mycoplasma species or in which they play a prominent role, and against which compounds of this invention are effective, are as follows:

Avian

M gallisepticum - Chronic respiratory diseases (air-sacculitis) of chickens and Turkeys

Bovine

M-bovis - Mastitis, respiratory disease and arthritis of cattle

M dispar - Calf pneumonia

Porcine

M suipneumoniae - Enzootic pneumonia of pigs

M hyorhinis )
M hyosynoviae ) - arthritis in pigs

Human

M pneumoniae - primary atypical pneumonia

Compounds of the present invention are particularly useful in the treatment of enzootic pneumonia in animals such as pigs, cattle and sheep, because they also have activity against the bacteria Bordetella bronchispetica, Pasteurella multocida and Haemophilus spp, which often cause respiratory complications in cases of this disease.

Accordingly the present invention also provides a pharmaceutical or veterinary composition which comprises a compound of formula (I) (hereinafter referred to as the "drug") together with a pharmaceutically or veterinary acceptable carrier or excipient.

The compositions may be formulated for administration by any route appropriate to the disease being treated. The compositions may be in the form of tablets, capsules, powders, granules, lozenges, and liquid or gel preparations, such as oral, topical or sterile parenteral solutions or suspensions.

Tablets and capsules for oral administration may be in unit-dosage form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; and disintegrants,

for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain aqueous or non aqueous vehicles (which may include edible oils), for example almond oil, fractionated coconut oil, oily esters such as glycerine, propylene glycol or ethyl alcohol and conventional additives such as suspending agents, for example sorbitol, syrup, methyl cellulose, glucose syrup, gelatin hydrogenated edible fats; emulsifying agents, for example lecithin, sorbitan monooleate, or acacia; preservatives, for example methyl or propyl p-hydroxybenzoate or sorbic acid and, if desired, conventional flavouring or colouring agents.

For topical application to the skin, the drug may be made up into a cream, lotion or ointment. Cream or ointment formulations that may be used for the drug are conventional formulations well known in the art, for example, as described in standard text books of pharmaceutics and cosmetics, such as Harry's Cosmeticology published by Leonard Hill Books, and the British Pharmacopoeia.

Suppositories will contain conventional suppository bases, eg cocoa-butters or other glyceride.

For parenteral administration, fluid unit dosage forms are prepared utilizing the drug and a sterile vehicle. The drug, depending on the vehicle and concentration used, can either be suspended or dissolved in the vehicle. In preparing solutions the drug can be

dissolved in water for injection and filter sterilized before filling into a suitable vial or ampoule and sealing. Advantageously, adjuvants such as a local anaesthetic, preservative and buffering agents can be dissolved in the vehicle. To enhance the stability the composition can be frozen after filling into the vial and water removed under vacuum. The dry lypophilized powder is then sealed in the vial. Parenteral suspensions are prepared in substantially the same manner except that the drug is suspended in the vehicle instead of being dissolved and sterilization cannot be accomplished by filtration. The drug can be sterilized by exposure to ethylene oxide before suspending in the sterile vehicle. Advantageously, a surfactant or wetting agent is included in the composition to facilitate uniform distribution of the drug.

For topical application to the ear, the drug may be made up into a solution or suspension in a suitable liquid carrier, such as water, glycerol, diluted ethanol, propylene glycol, polyethylene glycol or fixed oils.

For topical application to the eye, the drug is formulated as a solution or suspension in a suitable, sterile aqueous or non-aqueous vehicle. Adjuvants, for instance buffers such as sodium metabisulphite or disodium edetate; preservatives including bactericidal and fungicidal agents, such as phenylmercuric acetate or nitrate, benzalkonium chloride or chlorhexidine, and thickening agents such as hypromellose may also be included.

The dosage employed for compositions administered topically will, of course, depend on the size of the area being treated. For the ears and eyes each dose will typically be in the range from 10 to 100 mg of the drug.

Veterinary compositions for intramammary treatment of mammary disorders in animals, especially bovine mastitis, will generally contain a suspension of the drug in an oily vehicle.

The compositions may contain from 0.1% to 99% by weight, preferably from 10-60% by weight, of the drug. Where the compositions are in unit dosage form, each unit will preferably contain from 50-500 mg, of the drug. The dosage as employed for adult human treatment will preferably range from 100 mg to 3 g, per day, for instance 250 mg to 2 g, per day, depending on the route and frequency of administration.

Alternatively, the drug may be administered to animals as part of the total dietary intake. In this case the amount of drug employed may be less than 1% by weight of the diet and is preferably no more than 0.5% by weight. The diet for animals may consist of normal foodstuffs to which the drug may be added or it may be

included in a premix.

A suitable method for administering the drug to animals is to add it to the animals' drinking water. In this case a concentration of drug in the drinking water of about 5-500 μg/ml, for example 5-200 μg/ml, is suitable.

The present invention further provides a method for treating bacterial and/or mycoplasmal infections in human and non-human animals, which method comprises administering an effective, non-toxic amount of a compound of formula (I) as hereinbefore defined to a human or non human mammal suffering bacterial and/or mycoplasmal infection.

Alternatively, a pharmaceutical or veterinary composition, as hereinbefore described, is employed in the above method.

The invention will now be illustrated by the following Examples, which are not intended to limit the scope of the invention in any way.

The following abbreviations are used in the Examples:

| | |
|---|---|
| DMF | N,N-dimethylformamide |
| $MgSO_4$ | anhydrous magnesium sulphate |

EXAMPLE 1

p-Methylthiobenzyl monate A

(a) p-Methylthio benzyl alcohol

Sodium borohydride (1.2 g, 60 mmol) in ethanol (75 ml) was added dropwise to p-(methylthio)benzaldehyde in ethanol (25 ml) at 20°C and stirred for ½ h. The solution was evaporated under reduced pressure, and the residue partitioned between ether and sodium bicarbonate solution. The ether layer was dried (magnesium sulphate) and evaporated under reduced pressure to yield p-methylthiobenzyl alcohol (6.42 g, 69%); m.p. 36-38°C;

$^1$H nmr $\delta_H$ ($CDCl_3$) 7.15 (4H, s, aryl), 4.45 (2H, s, $CH_2$), 2.40 (3H, s, $SCH_3$).

(b) p-Methylthiobenzyl chloride

Thionyl chloride (3 ml, 41 mmol) was added dropwise to p-methylthiobenzyl alcohol (3.08 g, 20 mmol) in ether (25 ml) and stirred for ½ h. The reaction mixture was poured into water, extracted with ethyl acetate, dried (magnesium sulphate) and evaporated under reduced pressure to give p-methylthiobenzyl chloride (3.0 g, 87%);

$^1$H nmr $\delta_H$ ($CDCl_3$) 7.25 (4H, s, aryl), 4.55 (2H, s, $CH_2$), 2.50 (3H, s, $SCH_3$).

(c) p-Methylthiobenzyl monate A

Sodium monate A (3.65 g, 10 mmol) p-methylthiobenzyl chloride (1.72 g, 10 mmol) and dimethyl formamide (25 ml) were stirred at 20°C for 22 h. and then evaporated under

reduced pressure. The residue was taken up in ethyl acetate, washed with aqueous sodium bicarbonate, then brine, dried (magnesium sulphate), and evaporated under reduced pressure. The residual oil was purified by chromatography (15 g silicagel, 0 to 6% methanol in dichloromethane). Pure fractions were combined to give p-methylthiobenzyl monate A as a white solid (1.5 g, 31%); m.p. 76-79°C;

i.r. spectrum: $\nu_{max}$ (film)
3440, 1710, 1660 $cm^{-1}$;

U.V. spectrum: $\lambda_{max}$ (EtOH)
217.5 nm ($\varepsilon_m$ 20,098);

$^1H$ nmr: $\delta_H$ ($CDCl_3$)
7.29 and 7.24 (4H, ABq, aryl), 5.79 (1H, s, H2), 5.09 (2H, s, $CH_2$-1'), 2.48 (3H, s, $SCH_3$), 2.22 (3H, s, $CH_3$-15), 1.21 (3H, d, $CH_3$-14), 0.94 (3H,d, $CH_3$-17);

$^{13}C$ nmr: $\delta_C$ ($CDCl_3$)
166.4 (C1), 157.7 (C3), 138.6 (C1"), 133.3 (C4"), 128.9, 126.8 (C2", 3", 5", 6"), 117.3 (C2), 75.0 (C5), 71.3 (C13), 70.4 (C7), 69.0 (C6), 65.4 (C16), 65.2 (C1'), 61.3 (C11), 55.6 (C10), 42.9 (C12), 42.8 (C4), 39.6 (C8), 31.6 (C9), 20.8 (C14), 19.3 (C15), 15.9 (C7"), 12.6 (C17);

mass spectrum: m/e (relative intensity)
480 ($M^+$, 0.4%), 137 (100) (Found: $M^+$, 480.2218. $C_{25}H_{36}O_7S$ requires 480.2181).

<u>analysis</u>:

| | | |
|---|---|---|
| | Found: | C, 62.24; H, 7.31% |
| $C_{26}H_{36}O_7S$ | requires: | C, 62.50; H, 7.50% |

EXAMPLE 2

p- Methylsulphinylbenzyl monate A

m-Chloroperbenzoic acid (0.101 g, 0.5 mmol) in dichloromethane (20 ml) was added to a solution of p-methylthiobenzyl monate A (0.24 g, 0.5 mmol) and sodium bicarbonate (0.084 g, 0.5 mmol) in dichloromethane (20 ml) at $0^{o}C$. The reaction was stirred for 19 h, evaporated under reduced pressure and taken up in ethyl acetate/sodium bicarbonate solution. The organic layer was washed with brine, dried (magnesium sulphate) and evaporated under reduced pressure. The residue was chromatographed twice on silica (10 g) eluting with 0 to 6% methanol in methylene chloride. Pure fractions were combined and evaporated to yield p-methylsulphinylbenzyl monate A (104 mg, 42%);

i.r. spectrum: $\nu_{max}$ (film)
3440, 1710, 1640 $cm^{-1}$;

U.V. spectrum: $\lambda_{max}$ (EtOH)
222 nm (25,405);

$^{1}H$ nmr: $\delta_H$ ($CDCl_3$)
7.62 and 7.52 (4H, ABq, aryl), 5.81 (1H, s, H2), 5.16 (2H, s, $CH_2$-1'), 2.74 (3H, s, $SOC\underline{H}_3$), 2.21 (3H, s, $CH_3$-15), 1.23 (3H, d, $CH_3$-14), 0.94 (3H, d, $CH_3$-17);

$^{13}C$ nmr: $\delta_C$ ($CDCl_3$)
166.1 (C1), 158.8 (C3), 144.9 (C1"), 140.2 (C4"), 128.9, 123,9 (C2", 3", 5", 6"), 116.8 (C2), 75.0 (C5), 71.0 (C13), 70.4 (C7), 69.0 (C6), 65.5 (C16), 64.5 (C1'), 61.1 (C11), 55.6 (C10), 43.8, 43.1, 42.8 (C4, 12, 7"), 39.8 (C8), 31.8 (C9), 20.7 (C14), 19.3 (C15), 12.6 (C17).

EXAMPLE 3

p-Methylsulphonylbenzyl monate A

p-Methylthiobenzyl monate A (0.48 g, 1 mmol), and sodium bicarbonate (0.32 g, 4 mmol) in dichloromethane (20 ml) was cooled to $0^{o}C$. A solution of m-chloroperbenzoic acid (0.4 g, 2 mmol) in dichloromethane (20 ml) was added and stirred for 2 h at $0^{o}C$. The reaction mixture was allowed to warm to room temperature and stirred for 2 days. The reaction was evaporated under reduced pressure and then taken up in ethyl acetate/sodium bicarbonate solution. The organic layer was dried (magnesium sulphate), evaporated under reduced pressure and purified by chromatography (15 g silicagel, 0 to 6% methanol in dichloromethane) to yield p-methylsulphonylbenzyl monate A as a colourless oil (140 mg, 27%);

i.r. spectrum: $\nu_{max}$ (film)
3440, 1710, 1640 $cm^{-1}$;

U.V. spectrum: $\lambda_{max}$ (EtOH)
225 nm ($\varepsilon_m$ 23,605);

$^{1}H$ nmr: $\delta_H$ ($CDCl_3$)
7.95 and 7.57 (4H, ABq, aryl), 5.85 (1H, s, H2), 5.23 (2H, s, $CH_2$), 3.07 (3H, s, $SO_2CH_3$), 2.21 (3H, s, $CH_3$-15), 1.23 (3H, d, $CH_3$-14), 0.94 (3H, d, $CH_3$-17);

$^{13}C$ nmr: $\delta_C$ ($CDCl_3$)
166.0 (C1), 159.0 (C3), 143.0 (C1"), 140.1 (C4"), 128.6, 127.7 (C2", 3", 5", 6"), 116.7 (C2), 75.0 (C5), 71.3 (C13), 70.4 (C7), 69.0 (C6), 65.5 (C16), 64.2 (C1'), 61.2 (C11), 55.6 (C10), 44.5, 43.0, 42.8 (C4, 12, 7"), 39.7 (C8), 31.7 (C9), 20.8 (C14), 19.3 (C15), 12.7 (C17);

<u>mass spectrum</u>: m/e (relative intensity)
494 ($M^+-H_2O$, 0.4%) 227 (20), 186 (26), 157 (60), 107 (100) (Found: $M^+-H_2O$, 494.1940, $C_{25}H_{34}O_8S$ requires 494.1919);

<u>analysis</u>:

Found: C, 58.8, H, 6.9%
$C_{25}H_{36}O_9S$ requires: C, 58.6, H, 7.0%.

EXAMPLE 4

p-Methylsulphonylbenzyl monate A

p-Methylthiobenzyl chloride (2.59 g, 15 mmol) in dichloromethane (20 ml) was cooled to 0°C. m-chloroperbenzoic acid (6 g, 30 mmol) in dichloromethane (80 ml) was added to this solution and stirred for ½ h. The solution was warmed to room temperature and stirred for 18 h. The reaction mixture was then poured into sodium bicarbonate solution, and the organic layer dried (magnesium sulphate), and evaporated under reduced pressure. NMR indicated only 50% sulfone formation. The residue was therefore dissolved in dichloromethane (40 ml) and m-chloroperbenzoic acid (3 g, 15 mmol) added and stirred overnight. The reaction mixture was then filtered, the filtrate washed with aqueous sodium bicarbonate solution, dried (magnesium sulphate) and evaporated to yield p-methylsulphonylbenzyl chloride (2.52 g, 83%);

$^1$H nmr: $\delta_H$ ($CDCl_3$)
8.1 - 7.4 (4H, m, aryl), 4.65 (2H, s, $C\underline{H}_2$),
3.15 (3H, s, $SO_2C\underline{H}_3$).

p-Methylsulphonylbenzyl chloride (2.52 g, 12 mmol), sodium monate A (4.43 g, 12 mmol), and dimethyl formamide (70 ml) were stirred for 20 h at 20°C and then evaporated under reduced pressure,. The residue was taken up in ethyl acetate, washed with aqueous sodium bicarbonate, then brine, dried (magnesium sulphate) and evaporated under reduced pressure. The resulting residue was purified by chromatography (25 g silicagel, 0 to 4% methanol in dichloromethane). Pure fractions were combined to give a colourless oil, which crystallized from ether to yield p-methylsulphonylbenzyl monate A as a white solid (1.47 g, 24%); m.p. 104-106°C identical with Example 3.

EXAMPLE 5

m-Methylthiobenzyl monate A

3-Methylthiotoluene (0.58 g, 5 mmol), N-bromosuccinimide (0.534 g, 5 mmol), benzoyl peroxide (catalytic amount) and carbon tetrachloride (20 ml) were heated under reflux for 3 h. The reaction mixture was then cooled, filtered, washed with carbon tetrachloride and evaporated under reduced pressure to yield 3-methylthiobenzyl bromide (0.92, 84%).

Sodium monate A (1.8 g, 5 mmol), 3-methylthiobenzyl bromide (0.92 g, 5 mmol), and dimethyl formamide (20 ml) were stirred at 20°C for 18 h and then evaporated under reduced pressure. The residue was taken up in ethyl acetate, washed with aqueous sodium bicarbonate, then brine, dried (magnesium sulphate), and evaporated under reduced pressure. The resulting residue was purified by chromatography (15 g silicagel, 0 to 6% methanol in dichloromethane) to yield m-methylthiobenzyl monate A as a colourless oil (270 mg, 13%);

i.r. spectrum: $\nu_{max}$ (film)
3400, 1710, 1640 $cm^{-1}$;

U.V. spectrum: $\lambda_{max}$ (EtOH)
221 nm ($\varepsilon_m$ 19,340);

$^1H$ nmr: $\delta_H$ ($CDCl_3$)
7.30 - 7.00 (4H, m, aryl), 5.80 (1H, s, H2), 5.45 (2H, s, $CH_2$), 2.34 (3H, s, $SCH_3$), 2.22 (3H, s, $CH_3$-15), 1.21 (3H, d, $CH_3$-14), 0.94 (3H, d, $CH_3$-17);

$^{13}$C nmr: $\delta_C$ ($CDCl_3$)

165.8 (C1), 159.0 (C3), 138.9 (C1"), 134.8 (C3"), 130.8, 128.9, 128.1, 127.2 (C2", 4", 5", 6"), 116.9 (C2), 74.9 (C5), 71.2 (C13), 70.3 (C7), 69.0 (C6), 65.5 (C16), 63.6 (C1'), 61.2 (C11), 55.6 (C10), 43.0 (C12), 42.8 (C4), 39.6 (C8), 31.6 (C9), 21.3 (S$\underline{C}H_3$), 20.7 (C14), 19.3 (C15), 12.6 (C17);

mass spectrum: m/e (relative intensity)

480 ($M^+$, 2.20%), 327 (60), 309 (30), 227 (32), 137 (100) (Found: $M^+$, 480.2205, $C_{25}H_{36}O_7S$ requires 480.2179).

## Example 6

### m-Methylsulfinylbenzyl monate A

*m*-Chloroperbenzoic acid (0.050 g, 0.25 mmol) was added to a solution of *m*-methylthiobenzyl monate A (120 mg, 0.25 mmol) and sodium bicarbonate (0.042 g, 0.25 mmol) in dichloromethane (10 ml). The solution was stirred for 18 h at room temperature and then filtered. The filtrate was washed with aqueous sodium bicarbonate solution, the organic layer dried ($MgSO_4$) and evaporated under reduced pressure. The residue was purified by chromatography (8 g silicagel, 0 to 4% methanol in dichloromethane) to yield the title ester as a colourless oil (45 mg, 36%); $\nu_{max}$ (film) 3400, 1710, 1640, 1120 $cm^{-1}$; $\lambda_{max}$ (EtOH) 226 nm ($\varepsilon_m$25,860); $\delta_H$ ($CDCl_3$) 7.55 - 7.32 (4H, m, aryl), 5.88 (1H, s, H2), 4.96 (2H, ddd, $CH_2$), 2.45 (3H, s, $SOCH_3$), 2.23 (3H, s, $CH_3$-15), 1.25, 1.22 (3H, 2 x d, $CH_3$-14), 0.94, 0.90 (3H, 2 x d, $CH_3$-17); $\delta_C$ ($CDCl_3$) 164.7 (C1), 161.8 (C3), 140.1 (C3"), 139.9 (C1"), 132.7 (C6"), 129.3 (C5"), 124.8 (C2"), 121.7 (C4"), 115.6 (C2), 82.2, 82.1 (C1'), 74.9 (C5), 71.4 (C13), 70.5 (C7), 68.9, 68.7 (C6), 65.5 (C16), 61.3 (C11), 55.5, 55.3 (C10), 43.3, 43.1 (C4), 42.8 (C12), 39.7 (C8), 31.7 (C9), 23.0 (C7"), 21.4, 20.9 (C14), 19.8, 19.7 (C15), 12.6, 12.5 (C17); *m/e* (relative intensity, CI, $NH_3$) 497 ($MH^+$, 32%), 344 (58), 327 (100).

Example 7

m-Methylsulfonylbenzyl monate A

m-Thiocresol (3.7 g, 30 mmol) and sodium hydroxide (1.2 g, 30 mmol) were dissolved in ethanol (50 ml). To this solution were added methyl iodide (1.9 ml, 30 mmol) and benzene (50 ml) and stirred for 5 h. The reaction was then evaporated under reduced pressure, taken up in ethyl acetate, washed with aqueous sodium bicarbonate solution, dried ($MgSO_4$), and evaporated under reduced pressure (2.98 g, 72%); $\delta_H$($CDCl_3$) 7.40 - 6.92 (4H, m, aryl), 2.40 (3H, s, $SCH_3$), 2.30 (3H, s, $CH_3$).

3-Methylthiotoluene (1.38 g, 10 mmol) in dichloromethane (40 ml) was cooled to 0°C and m-chloroperbenzoic acid (4.04 g, 20 mmol) added and stirred for 20 h at 20°C. The reaction was then filtered, washed with aqueous sodium bicarbonate solution, dried ($MgSO_4$), and evaporated under reduced pressure. The product was shown by p.m.r. to be a mixture of sulfoxide and sulfone and was therefore dissolved in dichloromethane (40 ml) and m-chloroperbenzoic acid (2.02 g, 10 mmol) added and stirred at 20°C for 19 h. The reaction mixture was filtered, the filtrate was washed with aqueous sodium bicarbonate solution, dried ($MgSO_4$), and evaporated under reduced pressure; $\delta_H$($CDCl_3$) 8.15 - 7.20 (4H, m, aryl), 3.00 (3H, s, $SO_2CH_3$), 2.40 (3H, s, $CH_3$).

m-Methylsulphonyltoluene (0.85 g, 5 mmol), N-bromosuccinimide (0.90 g, 5 mmol), and benzoyl peroxide (100 mg) were heated at reflux in carbon tetrachloride (25 ml) for 4 h. The reaction mixture was allowed to cool, filtered, and the filtrate evaporated under reduced pressure. The residue was then purified

by chromatography (10 g silicagel, dichloromethane) to yield a pale yellow oil (1.0 g, 80%); $\delta_H(CDCl_3)$ 8.20 - 7.15 (4H, m, aryl), 4.50 (2H, s, $CH_2$), 3.0 (3H, s, $SOCH_3$).

A solution containing sodium monate A (1.83 g), m-methylsulfonylbenzyl bromide (1.25 g) and DMF (75 ml) was stirred overnight at room temperature and then evaporated under reduced pressure. The residue was taken up in ethyl acetate/brine and the organic phase washed with aqueous sodium bicarbonate and brine then dried ($MgSO_4$) and evaporated under reduced pressure. The resulting residue was purified by column chromatography (silica gel, eluting with 0-6% methanol in chloroform) to yield the title ester as a colourless oil (197 mgs, 8%); $\nu_{max}$ (film) 3440, 1710, 1640, 1300, 1220, 1140 $cm^{-1}$; $\lambda_{max}$ (EtOH) 223 nm ($\varepsilon_m$23,599); $\delta_H(CDCl_3)$ 7.90 (1H, s, H2"), 7.81 (1H, d, H4"), 7.60 - 7.54 (2H, m, H5",6"), 5.85 (1H, s, H2), 5.21 (1H, s, $CH_2$), 3.09 (3H, s, $CH_3$), 2.22 (3H, s, $CH_3$-15), 1.21 (3H, d, $CH_3$-14), 0.94 (3H, d, $CH_3$-17); $\delta_C(CDCl_3)$ 166.0 (C1), 159.0 (C3), 140.9 (C3"), 138.5 (C1"), 133.2 (C6"), 129.7 (C5"), 126.8 (C2"), 126.6 (C4"), 116.7 (C2), 74.9 (C5), 71.1 (C13), 70.3 (C7), 68.9 (C6), 65.4 (C16), 64.2 (C1'), 61.2 (C11), 55.6 (C10), 44.5 (C7"), 43.0 (C4), 42.8 (C12), 39.6 (C8), 31.6 (C9), 20.7 (C14), 19.3 (C15), 12.6 (C17); m/e (relative intensity, CI, $NH_3$) 530 ($MNH_4^+$, 1%), 513 ($MH^+$, 1), 327 (5), 204 (88), 188 (100).

Example 8

1-(p-Methylsulfinylphenyl)ethyl monate A

p-Methylthioacetophenone (2.49 g, 15 mmol) and sodium borohydride (0.3 g, 15 mmol) were stirred in ethanol (50 ml) for 45 min. The reaction mixture was evaporated under reduced pressure and the residue partitioned between ether and aqueous sodium bicarbonate solution. The organic layer was dried ($MgSO_4$) and evaporated under reduced pressure to yield the alcohol as a colourless oil (2.86 g, 100%); $\delta_H(CDCl_3)$ 7.20 (4H, s, aryl), 4.80 (1H, q, $-\overset{|}{\underset{|}{C}}H$), 2.40 (3H, s, $SCH_3$), 1.35 (3H, d, $CH_3$).

The alcohol (2.86 g, 15 mmol) and phosphorus tribromide (0.48 ml, 5 mmol) in benzene (15 ml) were heated at 80°C for 2½ h. The reaction mixture was evaporated under reduced pressure and purified by chromatography (10 g silicagel, dichloromethane) to yield a colourless oil, which crystallised in ether to yield a white solid; m.p. 61-63°C (1.53 g, 45%); $\delta_H(CDCl_3)$ 7.1 - 7.6 (4H, m, aryl), 5.25 (1H, q, $-\overset{|}{\underset{|}{C}}H$), 2.50 (3H, s, $SCH_3$), 2.05 (3H, d, $CH_3$).

1-(p-Methylthiophenyl)ethyl bromide (0.46 g, 2 mmol) and m-chloroperbenzoic acid (0.406 g, 2 mmol) was stirred in methylene chloride at 0°C for 3 h. The reaction mixture was evaporated under reduced pressure and the residue taken up in ethyl acetate. The organic layer was washed with aqueous sodium bicarbonate, dried ($MgSO_4$), and evaporated under reduced pressure. The residue was treated with sodium monate A (732 mgs) in DMF (30 ml) at room temperature overnight and the mixture evaporated under reduced pressure. The residue was taken up in ethylacetate/brine then dried ($MgSO_4$) and evaporated under reduced pressure. The resulting

residue was purified by column chromatography (silica gel, eluting with 0-6% methanol in chloroform) to yield title compound as a colourless oil (239 mg, 23%); $\nu_{max}$ (film) 3400, 1710, 1640, 1220, 1150 $cm^{-1}$; $\lambda_{max}$ (EtOH) 224 nm ($\epsilon_m$ 22,385); $\delta_H$($CDCl_3$) 7.63 and 7.53 (4H, ABq, aryl), 5.94 (1H, q, C<u>H</u>-1'), 5.83 (1H, s, H2), 2.74 (3H, s, COC<u>H</u>$_3$), 2.20 (3H, s, $CH_3$-15), 1.22 (3H, d, $CH_3$-14), 0.95 (3H, d, $CH_3$-17); $\delta_C$($CDCl_3$) 165.6 (C1), 158.5 (C3), 146.0, 145.9 (C1"), 144.1 (C4"), 127.2, 127.1, 123.9 (C2", 3", 5", 6"), 117.1, 117.0 (C2), 74.9 (C5), 70.8 (C13), 70.7 (C7), 70.3 (C1'), 68.9 (C6), 65.5 (C16), 61.0 (C11), 55.6 (C10), 43.6, 43.0, 42.7 (C7", 4, 12), 39.7 (C8), 31.7 (C9), 22.4 (C2'), 20.7 (C14), 19.2 (C15), 12.5 (C17); <u>m/e</u> (relative intensity 510 ($M^+$, 0.59%), 308 (8), 295 (18), 266 (52), 227 (43), 167 (100)
(Found: $M^+$ = 510.2266. $C_{26}H_{38}O_8S$ requires 510.2287).

Example 9

1-(p-Methylsulfonylphenyl)ethyl monate A

1-(p-Thiomethylphenyl)ethyl bromide (0.46 g, 2 mmol) and m-chloroperbenzoic acid (0.81 g, 4 mmol) were stirred in methylene chloride at 0°C for 6 h and left standing overnight. The reaction mixture was filtered and the filtrate evaporated under reduced pressure. The residue was taken up in ethyl acetate, washed with aqueous sodium bicarbonate, dried ($MgSO_4$) and evaporated under reduced pressure. The residue was treated with sodium monate A (732 mgs) in DMF (30 ml) and stirred at room temperature overnight and the mixture evaporated under reduced pressure. The residue was taken up in ethyl acetate/brine and the organic phase washed with aqueous sodium bicarbonate and brine then dried ($MgSO_4$) and evaporated under reduced pressure. The resulting residue was purified by column chromatography (silica gel, eluting with 0-6% methanol in chloroform) to yield the title compound as a colourless oil (220 mg, 21%); $\nu_{max}$(film) 3440, 1710, 1660, 1310, 1220, 1150 $cm^{-1}$; $\lambda_{max}$ (EtOH) 226 nm; ($\varepsilon_m$27,169); $\delta_H$($CDCl_3$) 7.9 and 7.55 (4H, ABq, aryl), 5.90 (1H, q, CH-1'), 5.80 (1H, s, H2), 3.05 (3H, s, $SO_2C\underline{H}_3$), 2.18 (3H, s, $CH_3$-15), 1.20 (3H, d, $CH_3$-14), 0.92 (3H, d, $CH_3$-17); $\delta_C$($CDCl_3$) 165.5 (C1), 158.7 (C3), 148.7, 148.6 (C1"), 139.5 (C4"), 127.6, 126.9 (C2", 3", 5", 6"), 116.9, 116.8 (C2), 74.8 (C5), 70.9 (C13), 70.6 (C7), 70.2 (C1'), 68.8 (C6), 65.4 (C16), 61.0 (C11), 55.6 (C10), 44.4 (C7"), 43.0, 42.7 (C12, 4), 39.6 (C8), 31.6 (C9), 22.4 (C2'), 20.7 (C14), 19.2 (C15), 12.5 (C17).

## BIOLOGICAL DATA

### a) Anti-Mycoplasmal Activity

The activity of the compounds of the Examples against various mycoplasma organisms was determined in Friis broth solidified with 0.9% agarose inoculated with $10^3$ to $10^5$ C.F.U. The MIC's (µg/ml) were recorded after 6 days incubation at $37^oC$ and are shown in Table 1.

### b) Veterinary Bacteria

The activity of the compounds of the Examples against various organisms important in veterinary infections was determined using two fold serial dilutions in Diagnostic Sensitivity Test Agar inoculated with $10^4$ organisms. The MIC's were determined after incubation for 18 hours at $37^oC$ and are shown in Table 2.

### c) Human Bacteria

The activity of the compounds of the Examples against various organisms important in human infections was determined by serial dilution in nutrient agar with 5% chocolated horse blood. The MIC's (µg/ml) were recorded after incubation for 18 hours at $37^oC$ and are shown in Table 3.

TABLE 1

MIC's (µg/ml) against Mycoplasma spp.

| ORGANISM | COMPOUND OF EXAMPLE NO. | | | | | | | |
|---|---|---|---|---|---|---|---|---|
| | 1 | 2 | 3 | 5 | 6 | 7 | 8 | 9 |
| M. suipneumoniae NB12 | 0.5 | 0.25 | 0.1 | 0.5 | 5.0 | 0.5 | 2.5 | 1.0 |
| M. suipneumoniae JF 435 | 0.5 | 0.25 | 0.25 | 0.5 | 10.0 | 1.0 | 2.5 | 1.0 |
| M. suipneumoniae HK(2) | 0.5 | 0.25 | 0.25 | 0.5 | 10.0 | 1.0 | 2.5 | 1.0 |
| M. suipneumoniae Str. 11 | 0.25 | 0.25 | 0.1 | 0.25 | 5.0 | 0.5 | 1.0 | 0.5 |
| M. suipneumoniae J2206/183b | 0.5 | 0.25 | 0.25 | 0.5 | 10.0 | 1.0 | 2.5 | 1.0 |
| M. suipneumoniae MS16 | 0.25 | 0.1 | 0.1 | 0.25 | 5.0 | 0.5 | 1.0 | 0.5 |
| M. suipneumoniae PW/C/210 | 0.25 | 0.1 | 0.1 | 0.25 | 5.0 | 0.5 | 1.0 | NG |
| M. suipneumoniae LABER | 0.25 | 0.25 | 0.1 | 0.25 | 5.0 | 0.5 | 2.5 | 0.5 |
| M. suipneumoniae UCD 1 | 0.5 | 0.25 | 0.25 | 0.5 | 10.0 | 1.0 | 2.5 | 1.0 |
| M. suipneumoniae TAM 6N | 0.5 | 0.5 | 0.25 | 0.5 | 10.0 | 1.0 | 2.5 | 1.0 |
| M. suipneumoniae ATCC 25095 | 0.5 | 0.25 | 0.1 | 0.5 | 5.0 | 0.5 | 2.5 | 0.5 |
| M. suipneumoniae NCTC 10110 | 0.5 | 0.25 | 0.25 | 0.5 | 10.0 | 1.0 | 2.5 | 1.0 |
| M. hyorhinis ATCC 23234 | 0.25 | 0.25 | 0.1 | 0.25 | 5.0 | 0.5 | 1.0 | 0.5 |
| M. hyorhinis ATCC 25021 | 0.25 | 0.1 | 0.05 | 0.25 | 5.0 | 0.5 | 1.0 | 0.5 |
| M. hyosynoviae ATCC 25591 | 0.05 | 0.05 | 0.025 | 0.5 | 5.0 | 0.25 | 0.5 | 0.25 |
| M. bovis NCTC 10131 | ≤0.01 | ≤0.01 | <0.01 | 0.025 | 0.25 | 0.025 | .025 | <.01 |
| M. bovigenitalium ATCC 14173 | 0.025 | 0.025 | ≤0.01 | 0.025 | 0.25 | 0.05 | 0.1 | 0.05 |
| M. dispar NCTC 10125 | 0.1 | 0.1 | 0.05 | 0.1 | 2.5 | 0.25 | 0.5 | 0.05 |
| M. gallisepticum S6 | 2.5 | 1.0 | 0.5 | 5.0 | >10 | 5.0 | 10 | 10 |
| M. pneumoniae ATCC 15492 | 1.0 | 1.0 | 0.25 | 5.0 | 5.0 | 2.5 | 10 | 0.5 |

TABLE 2

MIC's (μg/ml) against veterinary bacteria

| ORGANISM | COMPOUND OF EXAMPLE NO. | | | | | | | |
|---|---|---|---|---|---|---|---|---|
| | 1 | 2 | 3 | 5 | 6 | 7 | 8 | 9 |
| E. coli NCTC 10418 | >80 | >80 | 80 | >80 | >80 | >80 | >80 | >80 |
| E. coli E1 | >80 | >80 | >80 | >80 | >80 | >80 | >80 | >80 |
| S. dublin S7 | >80 | >80 | >80 | >80 | >80 | >80 | >80 | >80 |
| S. typhimurium S18 | >80 | >80 | >80 | >80 | >80 | >80 | >80 | >80 |
| Bord. bronchiseptica B08 | 20 | 40 | 20 | 10 | >80 | >80 | >80 | >80 |
| Bord. bronchiseptica BO9 | 5 | 5 | 2.5 | 2.5 | >80 | 20 | >80 | 80 |
| Past. multocida PA1 | .156 | .625 | .025 | 2.5 | 80 | 2.5 | 10 | 5.0 |
| Past. multocida PA2 | .156 | .625 | .156 | .625 | 20 | 1.25 | NG | 2.5 |
| Past. haemolytica PA5 | .312 | 5 | 5 | 10 | 80 | 10 | 80 | 40 |
| Erysipelothrix rhusiopathiae NCTC 8163 | 10 | 20 | 80 | 5 | >80 | >80 | >80 | 80 |
| Corynebacterium pyogenes CY1 | >80 | >80 | >80 | >80 | >80 | >80 | >80 | >80 |
| Staph. aureus B4 (pen. resistant) | .156 | 2.5 | 1.25 | .156 | 5.0 | 2.5 | 20 | 5.0 |
| Staph. aureus 152 (pen. sens.) | .156 | 1.25 | 1.25 | .156 | 5.0 | 2.5 | 5.0 | 5.0 |
| Staph. aureus Oxford | .156 | 1.25 | 1.25 | .156 | 5.0 | 2.5 | 20 | 10 |
| Strep. suis (group D) SPS11 | 2.5 | 2.5 | 2.5 | 1.25 | >80 | 20 | 80 | 40 |
| Strep. uberis SPU1 | .039 | .156 | .312 | .07 | 5.0 | .625 | .625 | .312 |
| Strep. dysgalactiae SPD1 | .156 | .312 | .156 | .07 | 20 | 1.25 | 2.5 | .625 |
| Strep. agalactiae SPA1 | .312 | .625 | .625 | .312 | 20 | 2.5 | >80 | 80 |
| B. subtilis ATCC 6633 | NG | NG | NG | NG | | | | |

TABLE 3

MIC's (µg/ml against human bacteria

| ORGANISM | COMPOUND OF EXAMPLE NO. 1 | 2 | 3 | 5 | 6 | 7 | 8 | 9 |
|---|---|---|---|---|---|---|---|---|
| E. coli NCTC 10418 | 100 | >100 | 100 | >100 | >100 | >100 | >128 | >128 |
| E. coli ESS | 1.0 | 0.5 | 0.5 | 5.0 | 25 | 2.5 | 4.0 | 4.0 |
| P. mirabilis 889 | 100 | 50 | 50 | >100 | >100 | >100 | >128 | >128 |
| K. aerogenes A | >100 | >100 | >100 | >100 | >100 | >100 | >128 | >128 |
| Ps. aeruginosa 10662 | >100 | >100 | >100 | >100 | >100 | >100 | >128 | >128 |
| Pasteurella multocida 1633 | 1.0 | 0.5 | 0.25 | 10 | 25 | 2.5 | 8.0 | 8.0 |
| Haemophilus influenzae Q1 | 0.25 | 0.05 | 0.05 | 1.0 | 2.5 | 0.5 | NG | NG |
| Haemophilus influenzae Wy21 | 0.25 | 0.1 | 0.1 | 1.0 | 2.5 | 0.5 | 1.0 | 0.5 |
| Neisseria catarrhalis 1502 | 0.25 | 0.5 | 0.25 | 0.5 | 12.5 | 1.0 | 4.0 | 2.0 |
| Bacillus subtilis 6633 | 0.25 | 1.0 | 0.5 | 0.1 | 12.5 | 2.5 | 4.0 | 1.0 |
| Corynebacterium xerosis 9755 | >100 | >100 | >100 | >100 | >100 | >100 | >128 | >128 |
| Sarcina lutea 8340 | >100 | >100 | >100 | >100 | >100 | >100 | NG | NG |
| Staph. aureus Oxford | 0.5 | 2.5 | 1.0 | 0.5 | 2.5 | 2.5 | 16 | 4.0 |
| Staph. aureus Russell | 0.5 | 2.5 | 2.5 | 1.0 | 12.5 | 5.0 | 32 | 16 |
| Staph. aureus W2827 | 0.5 | 2.5 | 2.5 | 0.5 | 12.5 | 5.0 | 32 | 16 |
| Strep. faecalis I | 100 | 50 | 50 | 100 | >100 | >100 | >128 | >128 |
| Strep. pyogenes R80/421-A | 0.1 | 0.5 | 0.25 | 0.1 | - | - | 2.0 | 1.0 |
| Strep. agalactiae 2788-B | 1.0 | 1.0 | 0.5 | 1.0 | 25 | 5.0 | 8.0 | 8.0 |
| Strep. spp. 64/848-C | 0.5 | 1.0 | 0.5 | 1.0 | - | - | 4.0 | 4.0 |
| Strep. pneumoniae CN33 | 0.5 | 1.0 | 0.5 | 1.0 | 25 | 1.0 | 8.0 | 4.0 |

CLAIMS

1. A compound of formula (I):

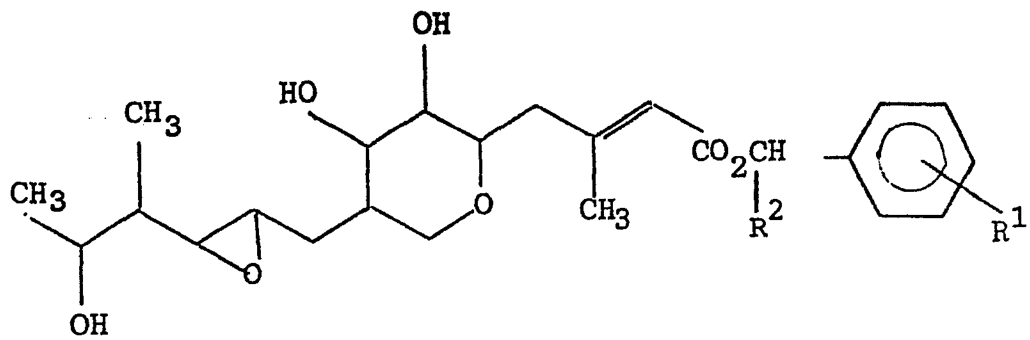

wherein $R^1$ is alkylthio, alkylsulphinyl or alkylsulphonyl, and $R^2$ is hydrogen or alkyl.

2. A compound as claimed in claim 1 wherein $R^1$ is selected from methylthio, ethylthio, methylsulphinyl, ethylsulphinyl, methylsulphonyl and ethylsulphonyl.

3. A compound as claimed in claim 1 or claim 2 wherein $R^2$ is hydrogen.

4. A compound as claimed in any one of claims 1 to 3 wherein $R^1$ is in the para-position.

5. A process for producing a compound of formula I, as defined in claim 1 which process comprises reacting a compound of formula (II):

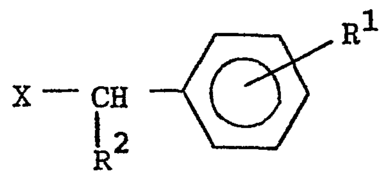

(II)

wherein X is a good leaving group with a reactive derivative of monic acid A.

6. A process as claimed in claim 5 wherein X is chlorine or bromine.

7. A process as claimed in claim 5 or claim 6 wherein the reactive derivative of monic acid A is the sodium salt thereof.

8. A pharmaceutical or veterinary composition comprising a compound of formula (I) as defined in claim 1 and a pharmaceutically or veterinarily acceptable carrier or excipient therefor.

9. A compound of formula (I) as defined in claim 1 for use in the treatment of the human or animal body.